# EUROPÄISCHE PATENTSCHRIFT

(11) **EP 1 789 108 B1**
(45) Veröffentlichungstag und Bekanntmachung des Hinweises auf die Patenterteilung: **15.09.2010**
(21) Anmeldenummer: 05784446.6
(22) Anmeldetag: 16.09.2005
(51) Int. Cl.: A61M 1/00, B08B 9/093

(54) **VORRICHTUNG ZUM ENTSORGEN VON FLÜSSIGKEITEN IM MEDIZINISCHEN BEREICH, INSBESONDERE ZUM ENTSORGEN VON DIALYSATEN**
DEVICE FOR DISPOSING OF LIQUIDS IN THE MEDICAL FIELD, ESPECIALLY FOR DISPOSING OF DIALYZATES
DISPOSITIF PERMETTANT L'ELIMINATION DE LIQUIDES DANS LE DOMAINE MEDICAL, EN PARTICULIER L'ELIMINATION DE DIALYSATS

(30) Priorität: 17.09.2004 AT 15662004
(43) Veröffentlichungstag der Anmeldung: 30.05.2007
(73) Patentinhaber: Hageneder, Konrad, 4643 Pettenbach (AT)
(72) Erfinder: Hageneder, Konrad, 4643 Pettenbach (AT)
(74) Vertreter: Hübscher, Helmut
(86) Internationale Anmeldenummer: PCT/AT2005/000373
(87) Internationale Veröffentlichungsnummer: WO 2006/029435

(56) Entgegenhaltungen:
- EP-A- 0 865 794
- US-A1- 2003 164 600

## Beschreibung

### Technisches Gebiet

Die Erfindung bezieht sich auf eine Vorrichtung zum Entsorgen von Flüssigkeiten im medizinischen Bereich, insbesondere zum Entsorgen von Dialysaten, mit einem auf Stützrädern fahrbar abgestützten, selbsttragenden Behälter und mit einer Austragseinrichtung für die über eine verschließbare Füllöffnung in den Behälter eingefüllte Flüssigkeit, wobei die Austragseinrichtung einen Druckluftanschluß im oberen Behälterbereich und eine vom Bodenbereich des Behälters ausgehende Austragsleitung umfaßt, die einen im Bereich einer Behälterwand aufsteigenden und einen daran anschließenden von der Behälterwand aus einer Ruhestellung in eine Auslaufstellung wegschwenkbaren Abschnitt aufweist.

### Stand der Technik

Zum Entsorgen von Dialysaten ist es bekannt (EP 0 865 794 B1), einen Behälter in Form eines Kanisters auf Stützrädern zu lagern und mit einer Austragseinrichtung zu versehen, die einen im oberen Behälterbereich vorgesehenen Druckluftanschluß sowie eine Auslauföffnung im Bodenbereich umfaßt, an die von außen ein Schlauch einer Austragsleitung angeschlossen ist. Diese Austragsleitung wird im Anschluß an den Schlauch durch ein Rohr gebildet, das einen zum Behälterboden senkrechten, um seine Achse verdrehbar in Klemmen auf einer Behälterwand gelagerten Rohrteil und einen dazu quer verlaufenden Rohrteil bildet, der an seinem freien Ende mit einer Verschlußkappe versehen ist. Zum Austragen von Dialysaten kann daher der verschwenkbare Abschnitt der Austragsleitung aus der gegen die Behälterwand eingeschwenkten Ruhestellung in eine von der Behälterwand abstehende Auslaufstellung verschwenkt werden, in der der Druckluftanschluß freigegeben wird, so daß die Behälterflüssigkeit durch die in den Behälter strömende Druckluft über die Austragsleitung aus dem Behälter verdrängt wird. Nachteilig ist allerdings, daß durch die Verdrehung des verschwenkbaren Abschnittes der Austragsleitung der Schlauchteil, der drehfest an der Auslauföffnung des Behälters angeschlossen ist, um seine Achse mitgedreht und folglich einer Torsionsbeanspruchung unterworfen wird, die bei längerem Gebrauch der Vorrichtung die Gefahr einer Überlastung des Schlauchteils mit sich bringt.

### Darstellung der Erfindung

Der Erfindung liegt somit die Aufgabe zugrunde, eine Vorrichtung zum Entsorgen von Flüssigkeiten im medizinischen Bereich, insbesondere zum Entsorgen von Dialysaten, der eingangs geschilderten Art mit einfachen konstruktiven Mitteln so auszugestalten, daß eine Überlastung der Austragsleitung ausgeschlossen werden kann, ohne auf einen ausschwenkbaren Leitungsabschnitt verzichten zu müssen.

Die Erfindung löst die gestellte Aufgabe dadurch, daß der aufsteigende Abschnitt der Austragsleitung innerhalb des Behälters vorgesehen und mit dem außerhalb des Behälters angeordneten, verschwenkbaren Abschnitt über eine Behälterdurchführung verbunden ist und daß der verschwenkbare Abschnitt der Austragsleitung in einer Anschlußmuffe außerhalb des Behälters drehbar lagert.

Da zufolge dieser Maßnahmen der aufsteigende Abschnitt der Austragsleitung innerhalb des Behälters verläuft, ist dieser Abschnitt der Austragsleitung vor äußeren Einflüssen geschützt, wobei durch eine entsprechende Behälterdurchführung die erforderliche Behälterdichtheit ohne Schwierigkeiten gewährleistet werden kann. Die drehbare Lagerung des verschwenkbaren Abschnittes der Austragsleitung wird ja erst außerhalb des Behälters mit Hilfe einer Anschlußmuffe erreicht. Die Abdichtung des gegenüber der Anschlußmuffe drehbar gehaltenen Abschnittes der Austragsleitung beruht somit auf einer bewährten Technik, die aufgrund der drehbaren Lagerung des verschwenkbaren Abschnittes in der Anschlußmuffe eine Übertragung von Torsionskräften und damit eine Torsionsbeanspruchung der Austragsleitung vermeidet. Erfindungsgemäße Vorrichtungen zum Entsorgen von Flüssigkeiten im medizinischen Bereich weisen daher keine von der Austragseinrichtung beeinträchtigte Standzeit auf.

Besonders vorteilhafte Konstruktionsverhältnisse ergeben sich, wenn der verschwenkbare Abschnitt aus einem zum Behälterboden im wesentlichen parallel verlaufenden, um seine Achse drehbar gelagerten Leitungsteil und einem daran anschließenden quer dazu verlaufenden Leitungsteil besteht, weil in diesem Fall der größte Abstand der Auslauföffnung des in die Auslaufstellung verschwenkten Abschnittes der Austragsleitung nicht von der durch die Behälterbreite bestimmten Länge des bodenparallelen Leitungsteils, sondern von dem dazu quer verlaufende Leitungsteil abhängt, der sich in der Ruhestellung der Höhe nach entlang der Behälterwand erstreckt, was aufgrund einer gegenüber der Behälterbreite wesentlich größeren Behälterhöhe einen die Handhabung erleichternden Abstand zwischen dem Behälter und der die Flüssigkeit aus dem Behälter aufnehmenden Entsorgungseinrichtung erlaubt.

Weist die Behälterwand eine vertiefte Aufnahme für den in die Ruhestellung verschwenkten Abschnitt der Austragsleitung auf, so kann auch der außerhalb des Behälters vorgesehene Abschnitt der Austragsleitung innerhalb des Behälterumrisses verlegt werden, was einen zusätzlichen Schutz der außerhalb des Behälters geführten Teile der Austragsleitung vor mechanischen Beschädigungen mit sich bringt.

### Kurze Beschreibung der Zeichnung

In der Zeichnung ist der Erfindungsgegenstand beispielsweise dargestellt. Es zeigen
- Fig. 1: eine erfindungsgemäße Vorrichtung zum Entsorgen von Flüssigkeiten im medizinischen Bereich in einer Ansicht auf die Breitseite des Behälters mit der Austragsleitung,
- Fig. 2: eine zum Teil aufgerissene Ansicht des Behälters auf seine Schmalseite und
- Fig. 3: einen Schnitt nach der Linie III-III der Fig. 1.

### Weg zur Ausführung der Erfindung

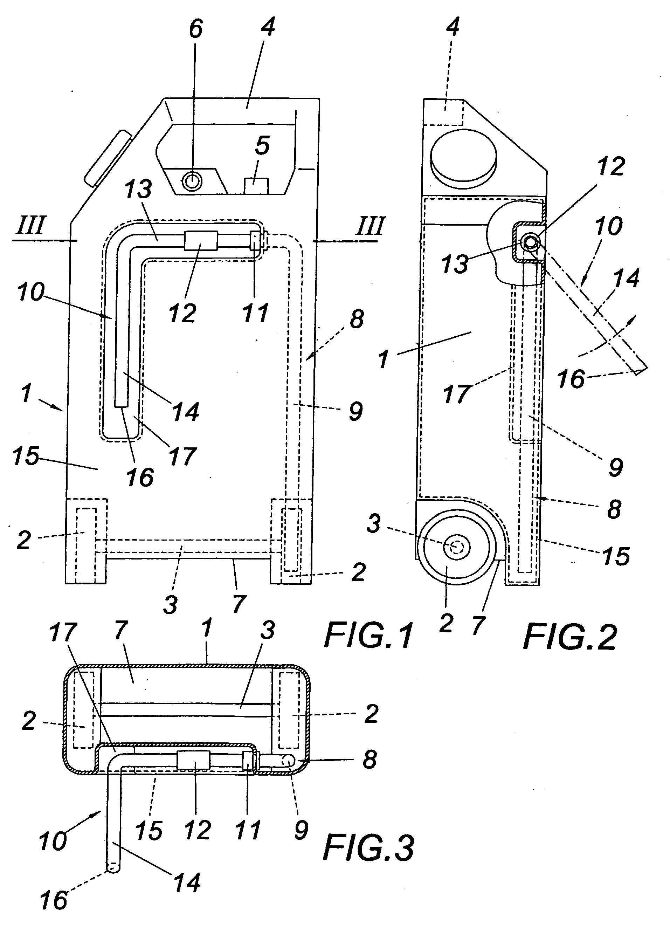

Die dargestellte Vorrichtung zum Entsorgen von Flüssigkeiten, insbesondere von Dialysaten, weist einen selbsttragenden Behälter 1 in Form eines Kanisters auf, der auf Stützrädern 2 verfahren werden kann, die auf einer gemeinsamen Achse 3 im Behälter 1 gelagert sind. Der Behälter 1, der mit einem Tragegriff 4 versehen ist, ist mit einer verschließbaren Füllöffnung 5 für die zu entsorgende Flüssigkeit versehen, die über eine Austragseinrichtung aus dem Behälter 1 entleert werden kann. Diese Austragseinrichtung umfaßt einen mit einem Rückschlagventil versehenen Druckluftanschluß 6 sowie eine vom Behälterboden 7 ausgehende Austragsleitung 8. Die Anordnung ist dabei so getroffen, daß der aufsteigende Abschnitt 9 der Austragsleitung 8 innerhalb des Behälters 1 vorgesehen ist und mit dem außerhalb des Behälters 1 angeordneten, verschwenkbaren Abschnitt 10 über eine Behälterdurchführung 11 in Verbindung steht. Der verschwenkbare Abschnitt 10 der Austragsleitung 8 ist dabei in einer Anschlußmuffe 12 drehbar gelagert, die sich auf der Außenseite der Behälterdurchführung 11 befindet. Der verschwenkbare Abschnitt 10 der Austragsleitung 8 setzt sich aus einem zum Behälterboden 7 im wesentlichen parallel verlaufenden Leitungsteil 13 und einem daran anschließenden, quer dazu verlaufenden Leitungsteil 14 zusammen. In der gegen die Behälterwand 15 eingeschwenkten Ruhestellung des schwenkbaren Abschnittes 10 verläuft der Leitungsteil 14 gemäß der Fig. 1 in Richtung der Behälterhöhe, was eine ausreichende Länge für den Leitungsteil 14 ermöglicht, um beim Ausschwenken des Abschnittes 10 einen entsprechenden Abstand der Auslauföffnung 16 vom Behälter 1 zu erreichen.

Damit der verschwenkbare Abschnitt 10 der Austragsleitung 8 in der Ruhestellung nicht in störender Weise den Umriß des Behälters 1 überragt, ist in der Behälterwand 15 eine vertiefte Aufnahme 17 für den in die Ruhestellung verschwenkten Abschnitt 10 der Austragsleitung 8 vorgesehen.

## Patentansprüche

1. Vorrichtung zum Entsorgen von Flüssigkeiten im medizinischen Bereich, insbesondere zum Entsorgen von Dialysaten, mit einem auf Stützrädern (2) fahrbar abgestützten, selbsttragenden Behälter (1) und mit einer Austragseinrichtung für die über eine verschließbare Füllöffnung (5) in den Behälter eingefüllte Flüssigkeit, wobei die Austragseinrichtung einen Druckluftanschluß (6) im oberen Behälterbereich und eine vom Bodenbereich (7) des Behälters ausgehende Austragsleitung (8) umfaßt, die einen im Bereich einer Behälterwand aufsteigenden (9) und einen daran anschließenden von der Behälterwand aus einer Ruhestellung in eine Auslaufstellung wegschwenkbaren Abschnitt (10) aufweist, **dadurch gekennzeichnet, daß** der aufsteigende Abschnitt (9) der Austragsleitung (8) innerhalb des Behälters (1) vorgesehen und mit dem außerhalb des Behälters (1) angeordneten, verschwenkbaren Abschnitt (10) über eine Behälterdurchführung (11) verbunden ist und daß der verschwenkbare Abschnitt (10) der Austragsleitung (8) in einer Anschlußmuffe (12) außerhalb des Behälters (1) drehbar lagert.

2. Vorrichtung nach Anspruch 1, **dadurch gekennzeichnet, daß** der verschwenkbare Abschnitt (10) aus einem zum Behälterboden (7) im wesentlichen parallel verlaufenden, um seine Achse drehbar gelagerten Leitungsteil (13) und einen daran anschließenden quer dazu verlaufenden Leitungsteil (14) besteht.

3. Vorrichtung nach Anspruch 1 oder 2, **dadurch, gekennzeichnet, da**ß die Behälterwand (15) eine vertiefte Aufnahme (17) für den in die Ruhestellung verschwenkten Abschnitt (10) der Austragsleitung (8) bildet.

## Claims

1. Device for disposing of liquids within the field of medicine, in particular for disposing of dialysates, having a self-supporting container (1) which is movably supported on support wheels (2), and having a discharge device for the liquid which is introduced into the container via a sealable filling opening (5), wherein the discharge device comprises a compressed air connection (6) in the upper container region and a discharge line (8) which issues from the floor region (7) of the container and comprises a portion (9) which rises in the region of a container wall and a portion (10) which is adjacent thereto and can be pivoted away from the container wall from a rest position to a run-out position, **characterised in that** the rising portion (9) of the discharge line (8) is provided within the container (1) and is connected to the pivotable portion (10), which is disposed outside the container (1), via a lead-through (11) in the container, and **in that** the pivotable portion (10) of the discharge line (8) is supported in a rotatable manner in a connection sleeve (12) outside the container (1).

2. Device as claimed in claim 1, **characterised in that** the pivotable portion (10) consists of a line part (13), which extends substantially in parallel with the container floor (7) and is mounted so as to be rotatable about its axis, and of a line part (14) which is adjacent thereto and extends transversely thereto.

3. Device as claimed in claim 1 or 2, **characterised in that** the container wall (15) forms a recessed receiver (17) for the portion (10) of the discharge line (8) which is pivoted into the rest position.

## Revendications

1. Dispositif permettant l'élimination de liquides dans le domaine médical, en particulier l'élimination de dialysats, avec un récipient (1) autoporteur, soutenu, de manière à pouvoir se déplacer, sur des roues d'appui (2), et avec un dispositif d'évacuation pour le liquide introduit dans le récipient par une ouverture de remplissage (5) obturable, le dispositif d'évacuation comprenant un raccordement d'air comprimé (6) dans la zone supérieure de récipient et une conduite d'évacuation (8), partant de la zone de fond (7) du récipient, présentant un tronçon (9), montant dans la zone d'une paroi de récipient, et un tronçon (10) s'y raccordant, susceptible d'être écarté de la paroi de récipient par pivotement, en passant d'une position de repos à une position d'évacuation, **caractérisé en ce que** le tronçon (9) montant de la conduite d'évacuation (8) est prévu à l'intérieur du récipient (1), et relié, par un passage de récipient (11), au tronçon (10) pivotant, disposé à l'extérieur du récipient (1), et **en ce que** le tronçon (10) pivotant de la conduite d'évacuation (8) est monté en palier permettant une rotation dans un manchon de raccordement (12), à l'extérieur du récipient (1).

2. Dispositif selon la revendication 1, **caractérisé en ce que** le tronçon (10) pivotant est composé d'une partie de conduite (13), s'étendant sensiblement parallèlement au fond de récipient (7), montée à rotation autour de son axe, et d'une partie de conduite (14) s'y raccordant, s'étendant perpendiculairement à celle-ci.

3. Dispositif selon la revendication 1 ou 2, **caractérisé en ce que** la paroi de récipient (15) forme un logement (17) creusé en profondeur, pour le tronçon (10), pivoté à la position de repos, de la conduite d'évacuation (8).
